# EUROPÄISCHE PATENTSCHRIFT

(11) **EP 0 396 874 B1**
(45) Veröffentlichungstag und Bekanntmachung des Hinweises auf die Patenterteilung: **26.01.1994**
(21) Anmeldenummer: 90104863.7
(22) Anmeldetag: 15.03.1990
(51) Int. Cl.: A61M 5/31, A61B 17/34, A61B 8/08

(54) **Spritze zum dopplersonographisch unterstützten Punktieren**
Syringe for Doppler sonography-guided puncture
Seringue pour jonction guidée par sonographie Doppler

(30) Priorität: 21.03.1989 DE 3909140
(43) Veröffentlichungstag der Anmeldung: 14.11.1990
(73) Patentinhaber: ISOTOPEN-TECHNIK DR. SAUERWEIN GMBH, D-42781 Haan (DE)
(72) Erfinder: Olivier, Lucien C. Dr., D-4300 Essen 13 (DE)
(74) Vertreter: König, Reimar, Dr.-Ing.

(56) Entgegenhaltungen:
- EP-A- 0 260 953
- DE-A- 2 142 642
- US-A- 3 556 079

## Beschreibung

Die Erfindung bezieht sich auf eine Spritze und ein Verfahren zum ultraschallunterstützten Punktieren arterieller und venöser Gefäße mit einem Empfänger und einem Kolben.

In der Intensivmedizin, aber auch bei vielen operativen Verfahren ergibt sich häufig die Notwendigkeit, in die großen Venen des Körpers Katheter zur Applikation von Flüssigkeiten und beispielsweise herzwirksamen Medikamenten einzuführen. Ebenso werden am arteriellen Gefäßsystem beispielsweise an der Arteria radialis des Handgelenks oder der Arteria femoralis der Leiste Katheter angelegt, um eine direkte Blutdruckmessung, aber auch Blutabnahme zur Blutgasbestimmung im arteriellen oxigenierten Blut durchzuführen. Beide Katheterlokalisationen, gleichviel ob sie arteriell oder venös liegen, sind Standardverfahren nicht nur chirugischer Kliniken. Punktionen großer Gefäße werden allerdings auch bei diagnostischen Verfahren, beispielsweise speziellen Katheteruntersuchungen in der Angiographie in großer Zahl durchgeführt.

Besonders die Punktion der Vena subclavia unter dem Schlüsselbein, aber auch die Punktion der Vena jugularis interna an den beiden Halsseiten ist belastet mit den verschiedensten Komplikationen, die zumeist durch frustrane und wiederholte Punktionsversuche bedingt sind. So kann es zum sogenannten Pneumothorax, zu Verletzungen der Halsschlagader, zu Hämatombildungen und anderen schweren iatrogenen Schäden kommen. Bei der Punktion der Arteria radialis oder auch der Arteria femoralis sind Gefäßschäden durch mehrfache blinde Punktionen bis hin zu einem Verschluß des betroffenen Gefäßes möglich. Außerdem führen frustrane Punktionsversuche zu einem Zeitverlust, der beispielsweise bei einer Notfallversorgung oder auch der Vorbereitung einer elektiven Operation unnötig ist.

Um iatrogene Schäden bei der Punktion großer Venen zu vermeiden, ist es bekannt, die Lage und den Verlauf des zu punktierenden Gefäßes sonographisch zu bestimmen. Dabei dient die bildgebende Sonographie zur Lokalisation beispielsweise der Vena jugularis interna des Halses. Entscheidender Nachteil dieser an sich praktikablen Methode zur Vermeidung iatrogener Schäden bei der Punktion großer Venen ist jedoch der apparative Aufwand, der dazu geführt hat, daß diese einfache und elegante Methode trotz der weiten Verbreitung bildgebender Sonographie-Geräte allgemein nicht benutzt wird.

Um die Schwierigkeiten bei der Gefäßlokalisation zu vermeiden und das Risiko von Komplikationen zu verringern, ist es auch bekannt, die Lage und den Verlauf des zu punktierenden Gefäßes mit Hilfe der Ultraschall-Doppler-Sonographie festzustellen. Dies geschieht mit Hilfe einer Sonde, deren Schallkopf auf das zu punktierende Gefäß ausgerichtet wird und eines mit einer üblichen Spritze mechanisch verbundenen externen Empfängers. Der Empfänger ist dabei in der Verlängerung der Nadelachse parallel zum Gehäuse der Spritze angeordnet, die über eine besondere Substanzleitung mit der Nadel verbunden ist. Der Ultraschallsender arbeitet normalerweise mit einer Frequenz von 4 bis 8 MHz.

Die Schallwellen werden von den sich in dem Gefäß bewegenden Blutkörperchen reflektiert und dabei aufgrund des Dopplereffekts verzerrt. Der Empfänger nimmt die reflektierten Schallwellen auf und gibt über einen Lautsprecher ein charakteristisches, bei Arterien ein pulssynchrones zischendes und bei Venen ein atemabhängiges heulendes Geräusch ab. Da sich das Geräusch mit der Annäherung an das gesuchte Gefäß verstärkt, bietet die Ultraschall-Doppler-Sonographie eine einfache Möglichkeit, Gefäße zu lokalisieren um die Fließrichtung des Blutes zu bestimmen, da bei in Richtung auf den Sender fließendem Blut die Schallwellen mit höherer und bei vom Sender wegfließendem Blut mit niedriger Frequenz reflektiert werden.

Probleme ergeben sich jedoch bei der Handhabung, da einerseits die Sonde plaziert und unabhängig davon die Nadel der mit dem Empfänger verbundenen Spritze in das Gewebe eingeführt werden muß. Das erfordert zumeist eine die Sonde haltende bzw. plazierende Hilfskraft, zumindest aber beide Hände des Untersuchenden. Außerdem handelt es sich bei der mit dem Empfänger verbundenen Spritze um eine verhältnismäßig komplizierte und wenig kompakte Sonderanfertigung, die sich zufolge ihrer Sperrigkeit und der ungünstigen Schwerpunktlage nur schwierig handhaben läßt. Eine Notfallversorgung ist daher mit Spritzen dieser Art nicht möglich.

Ein weiterer, beiden Verfahren gemeinsamer Nachteil besteht darin, daß außer der eigentlichen Spritze auch die Sonde und - bei der Doppler-Sonographie - der Empfänger sterilisiert oder steril verpackt sein müssen, was nicht nur aufwendig, sondern auch mit einer Gefährdung der elektrischen Bauteile verbunden ist.

In der europäischen Offenlegungsschrift 0 260 953 ist eine Spritze zum dopplersonographisch unterstützten Punktieren arterieller und venöser Gefäße mit einem Empfänger und einem Kolben beschrieben, bei dem der Empfänger in das Kanülenrohr integriert ist. Vom Kanülenrohr führt eine elektrische Verbindung zu einem nicht dargestellten Hochfrequenzsender, einem Doppler-Gerät und einem nachgeschalteten Lautsprecher. Bei der verwendeten Kanüle handelt es sich um eine komplizierte und wenig kompakte Sonderanfertigung, die sich daher nur schwierig handhaben läßt.

Der Erfindung liegt daher die Aufgabe zugrunde, eine Spritze mit einfachem Aufbau zu schaffen, die sich ebenso handhaben läßt wie übliche Normspritzen beim blinden Punktieren.

Die Lösung dieser Aufgabe besteht darin, daß bei einer Spritze der eingangs erwähnten Art der Empfänger, vorzugsweise aber auch der Sender erfindungsgemäß im Kolben angeordnet sind. Die Anordnung des Senders im Kolben einer handelsüblichen Spritze macht ein besonderes Plazieren des Senders überflüssig und gewährleistet, daß sich der Sender stets in einer optimalen Lage bezüglich des zu lokalisierenden Gefäßes befindet, ohne daß eine Hilfsperson oder eine zweite Hand erforderlich ist. Ebenso wie der Sender kann auch der Empfänger im Kolben angeordnet sein, um auf diese Weise eine besonders kompakte Spritze zu gewährleisten, die bei einem im Kolben angeordneten Lautsprecher das Suchgeräusch unmittelbar abgibt oder aber über eine im Kolben angeordnete Induktionsspule mit einem Außenlautsprecher gekoppelt ist.

Der Kolben kann ein mehrteiliges Gehäuse besitzen, um eine leichte Montage zu ermöglichen; er kann in einer sterilen Einmalhülse angeordnet sein und so in einen üblichen Spritzenzylinder eingeführt werden. Auf diese Weise läßt sich das System unter Verwendung üblicher Spritzenzylinder steril und sicher handhaben. Lediglich die sterile Einmalhülle des Kolbens wird nach jedem Gebrauch verworfen.

Eine wesentliche Erleichterung beim Lokalisieren des gesuchten Gefäßes ergibt sich, wenn mit Hilfe des Kolbens nach der Penetration der Haut und des subkutanen Fettgewebes eine geringe Menge einer in der Spritze befindlichen physiologischen Kochsalzlösung injiziert wird, um ein direktes Ankoppeln des Ultraschalls an die das gesuchte Gefäß umgebenden Weichteilen zu erreichen. Auf diese Weise gelingt es, auch ein in tieferen Schichten liegendes Gefäß zielsicher aufzufinden und zu punktieren. Sobald die Nadel der Spritze in das Gefäß eingedrungen ist, läßt sich wie im Falle einer blinden Gefäßpunktion Blut abzapfen und/oder nach dem Entfernen der erfindungsgemäßen Spritze und deren Hohlnadel aus einer Verweilkanüle in üblicher Weise ein intraarterieller oder intravenöser Katheter anlegen. Im Gegensatz zu der üblichen blinden Punktionstechnik kann die akurate Lage der Verweilkanüle durch zwischenzeitliche Kontrolle des Dopplersignals überprüft werden. Bei erschwertem Vorschieben eines Katheters über diese Verweilkanüle läßt sich so eine Fehllage der Kanüle als Ursache einer häufigen Behinderung bei der Anlage von Gefäßkathetern ausschließen.

Durch Verwendung mehrerer Quarzsender und ebenso vieler Quarzempfänger ist gewährleistet, daß während der Punktion stets ein Quarzpaar über der Auslaßöffnung zur Punktionsnadel zu liegen kommt.

Die Erfindung wird nachfolgend anhand eines in der Zeichnung dargestellten Ausführungsbeispiels des näheren erläutert. In der Zeichnung zeigen:

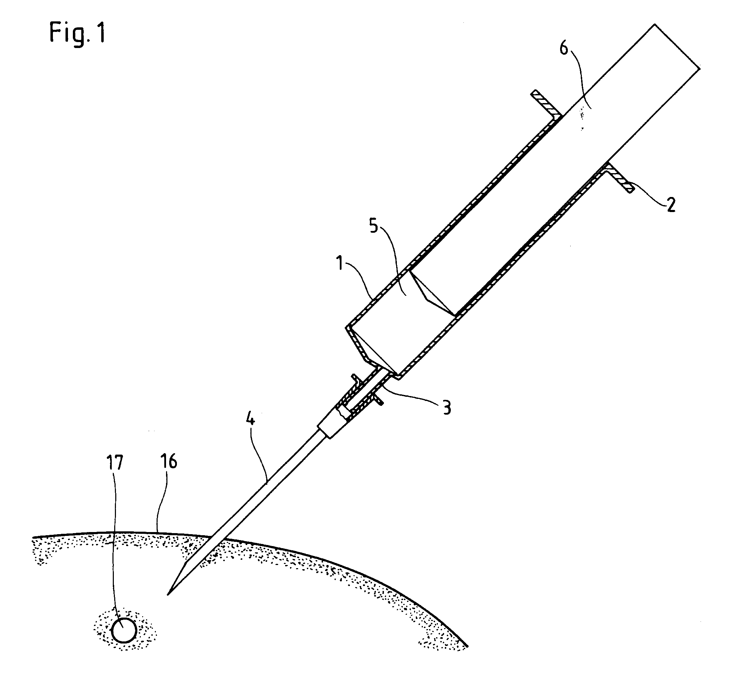

- Fig. 1: eine herkömmliche Spritze mit einem dopplersonographischen Kolben beim Lokalisieren eines Gefäßes und

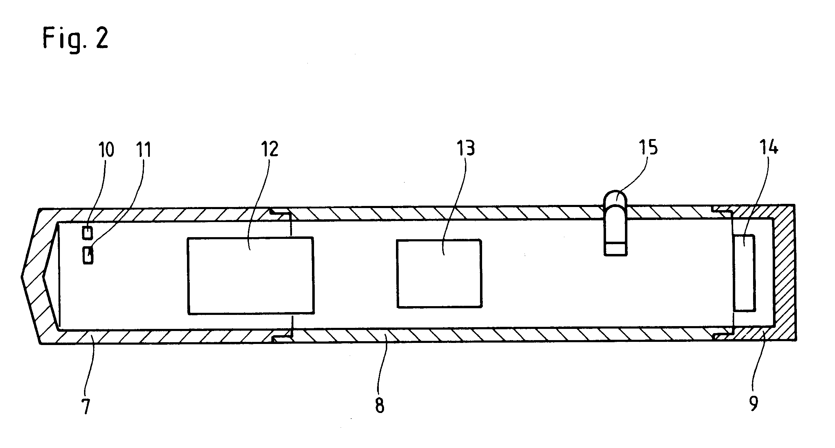

- Fig. 2: einen Längsschnitt durch den Kolben der Spritze nach Fig. 1.

Die erfindungsgemäße Spritze besteht aus einem üblichen Spritzenzylinder 1 mit einem Greifansatz 2 und einem Rodenansatz 3 als Aufnahme für eine Punktionsnadel 4 oder eine Verweilkanüle. Im Innern 5 der Spritze befindet sich ein verschiebbarer Kolben 6. Dieser Kolben besteht aus einem vorderen Teil 7, einem Mittelstück 8 und einer Kappe 9; er enthält im vorderen Teil 7 mindestens einen Quarzsender 10 und ebenso viele Quarzempfänger 11, die über nicht dargestellte Leitungen mit einer Sender- und Empfängerschaltung 12 verbunden sind. Eine vorzugsweise wiederaufladbare Spannungsquelle 13 dient der Stromversorgung. Von der Schaltung 12 verlaufen nicht dargestellte Leitungen zu einem im rückwärtigen Teil des Kolbens befindlichen Lautsprecher 14. Um den Sender 10 und den Empfänger 11 nicht dauernd in Betrieb zu halten, ist im Gehäuse des Kolbens 6 ein Tastschalter 15 angeordnet, der beim Einwärtsdrücken den Sender und gleichzeitig auch den Empfänger einschaltet.

Die Nadel 4 wird durch die Haut 16 in den Bereich des zu lokalisierenden Gefäßes 7 geführt. Alsdann wird durch leichtes Einwärtsdrücken des Kolbens 6 eine im Innern 5 der Spritze befindliche 9%-ige Kochsalzlösung injiziert, um eine bessere Fortleitung des von dem Sender 10 stammenden und von dem Gefäß 17 reflektierten transkutanen Dopplersignals zu erreichen.

Mit Hilfe der erfindungsgemäßen Spritze lassen sich auch bei schwierigen anatomisch-topographischen Verhältnissen und nach frustranen blinden Punktionsversuchen zu punktierende Gefäße mühelos lokalisieren und damit Fehlpunktionen sicher vermeiden. Dabei eignet sich die erfindungsgemäße Spritze bei Weglassung der Nadel oder auch nur der Kolben auch als Sonde zum Feststellen des anatomisch-topographischen Befundes, um auf diese Weise zunächst den Verlauf des Gefäßes festzustellen und einen möglichst günstigen Zugangsweg für die Nadel zu ermitteln. Bei entsprechend hoher Schallintensität eignet sich die Spritze oder auch nur der Kolben auch zur intraluminalen und periluminalen Doppler-Sonographie.

## Patentansprüche

1. Spritze zum dopplersonographisch unterstützten Punktieren arterieller und venöser Gefäße (17) mit einem Empfänger (11) und einem Kolben (6), dadurch gekennzeichnet, daß der Empfänger (11) in dem Kolben (6) angeordnet ist.

2. Spritze nach Anspruch 1, dadurch gekennzeichnet, daß in dem Kolben (6) ein Ultraschall-Sender (10) angeordnet ist.

3. Spritze nach Anspruch 1 oder 2, gekennzeichnet durch mindestens einen Quarzsender (10) und ebensoviele Quarzempfänger (11).

4. Spritze nach einem der Ansprüche 1 bis 3, dadurch gekennzeichnet, daß in dem Kolben (6) ein Lautsprecher (14) angeordnet ist.

5. Spritze nach einem der Ansprüche 1 bis 3, dadurch gekennzeichnet, daß der Empfänger (11) über eine im Kolben angeordnete Spule induktiv mit einem Außenlautsprecher gekoppelt ist.

6. Spritze nach einem der Ansprüche 1 bis 5, dadurch gekennzeichnet, daß im Kolben (6) ein Tastschalter (15) angeordnet ist.

7. Spritze nach einem oder mehreren der Ansprüche 1 bis 6, gekennzeichnet durch ein mehrteiliges Kolbengehäuse (7,8,9).

8. Spritze nach einem oder mehreren der Ansprüche 1 bis 7, gekennzeichnet durch eine sterile Einmalhülle.

## Claims

1. Syringe for Doppler sonographically aided penetration of arterial and venous vessels (17) comprising a receiver (11) and a plunger (6), characterised in that the receiver (11) is located in the plunger (6).

2. Syringe according to claim 1, characterized in that an ultrasonic transmitter (10) is located in the plunger (6).

3. Syringe according to claim 1 or claim 2, characterised by at least one quartz transmitter (10) and a corresponding number of quartz receivers (11).

4. Syringe according to any one of claims 1 to 3, characterised in that a loudspeaker (14) is located in the plunger (6).

5. Syringe according to any one of claims 1 to 3, characterised in that the receiver (11) is coupled inductively to an external loudspeaker by way of a coil located in the plunger.

6. Syringe according to any one of claims 1 to 5, characterised in that a momentary-contact switch (15) is located in the plunger (6).

7. Syringe according to one or more of claims 1 to 6, characterised by a multipart plunger housing (7, 8, 9).

8. Syringe according to one or more of claims 1 to 7, characterized by a sterile disposable sleeve.

## Revendications

1. Seringue pour la jonction guidée par sonographie Doppler d'artères et de veines (17), comportant un récepteur (11) et un piston (6), caractérisée en ce que le récepteur (11) est disposé dans le piston (6).

2. Seringue selon la revendication 1, caractérisée en ce que le piston (6) comporte un émetteur d'ultrasons (10).

3. Seringue selon la revendication 1 ou 2, caractérisée par au moins un émetteur à quartz (10) et par un nombre identique de récepteurs à quartz (11).

4. Seringue selon une quelconque des revendications 1 à 3, caractérisée en ce que le piston (6) comporte un haut-parleur (14).

5. Seringue selon une quelconque des revendications 1 à 3, caractérisée en ce que le récepteur (11) est couplé par induction à un haut-parleur externe via une hobine disposée dans le piston.

6. Seringue selon une quelconque des revendications 1 à 5, caractérisée en ce que le piston (6) comporte un commutateur à effleurement (15).

7. Seringue selon une ou plusieurs des revendications 1 à 6, caractérisee par un bâti (7, 8, 9) de piston en plusieurs parties.

8. Seringue une ou plusieurs des revendications 1 à 7, caractérisée par une gaine stérile à usage unique.
